# EUROPEAN PATENT SPECIFICATION

(11) **EP 0 794 785 B1**
(45) Date of publication and mention of the grant of the patent: **11.07.2001**
(21) Application number: 95938512.1
(22) Date of filing: 30.11.1995
(51) Int. Cl.: A61K 31/66, A61K 31/415, A61K 38/10, A61K 38/18

(54) **GROWTH FACTOR EXPRESSION IN HUMAN PREGNANT CERVIX**
WACHSTUMFAKTOEREN UND DEREN EXPRESSIONEN BEIM SCHWANGEREN GEBÄRMUTTERHALS
EXPRESSION DU FACTEUR DE CROISSANCE DANS LE COL UTERIN

(30) Priority: 30.11.1994 GB 9424232
(43) Date of publication of application: 17.09.1997
(73) Proprietor: THE UNIVERSITY OF BIRMINGHAM, Edgbaston, Birmingham B15 2TT (GB); Ahmed, Asif Syed, Birmingham B5 7SW (GB)
(72) Inventor: AHMED, Asif, Syed, Edgbaston, Birmingham B5 7SW (GB)
(74) Representative: Pearce, Anthony Richmond
(86) International application number: GB9502801
(87) International publication number: WO9616662

(56) References cited:
- CLIN. OBST. GYNAECOL., vol. 13, no. 2, June 1986 LONDON PHILADELPHIA TOKYO, pages 215-229, XP 000565915 G M STIRRAT & T A THOMAS 'Prescribing for labour'
- File Medline, Abstract no. 8823601, 1988 & ACTA EUROPAEA FERTILITATIS, vol. 19, no. 1, 1988 pages 33-36, DI LIETO ET AL. 'The morphological characteristics and ultrastructural aspects of cervical ripening induced by sulprostone'
- REVUE FRANÇAISE DE GYN COLOGIE ET D'OBST TRIQUE, vol. 81, no. 3, 1986 PARIS, pages 137-142, XP 000565914 C MICHELET ET AL. 'La maturation artificielle du col gravide par action locale de leucocytes maternels'
- AM. J. REPROD. IMMUNOL. MICROBIOL. , vol. 16, no. 3, March 1988 NEW YORK, pages 123-132, XP 000565913 J A MCGREGOR ET AL. 'Preterm birth and infection; pathogenic possibilities'
- J. REP. FERT., vol. 101, no. 2, July 1994 pages 459-466, XP 000564921 A AHMED ET AL. 'Functional platelet-activating factor receprtors linked to inositol lipid hydrolysis, calcium mobilization and tyrosine kinase activity in the human endometrial HEC_1B cell line '
- J. REP. FERT. ABSTRACTS, vol. , no. 13, 1994 page 3 XP 000564919 A AHMED ET AL. 'Functional platelet-activating factor receprtors linked to calcium mobilization and tyrosine kinase activity inhuman endometrial HEC-1B cell line'
- PLACENTA, vol. 16, no. 6, September 1995 page a42 XP 000564927 X. F. LI ET AL. 'Localisation of vascular endothelial growth factor and its receptor in pregnancy '
- 'Entry 8877: Sulprostone' 1983 , MERCK & CO. , RAHWAY, N. J. , USA see page 1292
- J REP FERT ABSTRACTS, no. 14, December 1994 page 6 XP 000564918 P SISI ET AL. 'Expression and localisation of vascular permeability factor in human cervix'
- BIOLOGY OF REPRODUCTION, vol. 51, no. 3, September 1994 pages 524-530, XP 000565923 D S CHARNOCK-JONES ET AL. 'Vascular endothlial growth factor receptor localisation and activation in human trophoblast and choriocarcinoma cells'

## Description

This invention relates to growth factor expression in cervical treatment and is more particularly concerned with agents for controlling softening or ripening of the cervix as a means of managing labour either to delay cervical ripening in order to delay delivery or to hasten cervical ripening in order to assist delivery.

### Background Art

Pre-term birth accounts for about 6-8% of all pregnancies and is responsible for 75% of all perinatal deaths. Currently, pre-term birth can be delayed for a few days by administering a muscle relaxing agent which serves to relax the myometrium. However, there is a need for agents which can delay the onset of labour for longer periods to avoid children being born very prematurely. Additionally, many full term deliveries are made difficult because, whilst there is strong myometrial activity, there is insufficient cervical ripening. Thus, there is a need to be able to control cervical ripening in a similar way to that in which myometrial activity can be controlled so that further control over pre-term and difficult full- or post-term deliveries can be exercised.

Di Lieto et al (File Medline Abstract No. 8823601, 1988 and Acta Europaea Fertilitatis 19(1), p33-36, 1988) analysed cervical tissue biopsies from fifteen pregnant women seeking second trimester elective abortions both before and after systemic sulprostone administration. Results indicated an increase in vascularity, suggesting that sulprostone can be useful to prime uterine cervix.

G.M. Stirrat et al (Clin. Obst. Gynaecol. 13(2), p215-229, 1986) review the use of several drugs in the manipulation of labour. For example, PGE₂ is used to ripen an unfavourable cervix or induce labour with a favourable cervix. Intravaginal relaxin and oestradiol also ripen the cervix, and oxytocin is known to induce labour.

The present invention has been made as a result of investigation of the mechanism which leads to ripening of the cervix during pregnancy. The cervix has commonly been regarded as serving a purely passive role in which it softens or ripens during pregnancy to facilitate passage of the foetus during delivery. It is known that prostaglandins and oestrogen have a part to play in the changes in cervical properties which take place during pregnancy, but the mechanism by which they act is poorly understood.

### Broad Disclosure of the Invention

During our investigation, it has been found that cervical ripening is dependent upon vascular permeability within the cervix.

In one of its aspects, the present invention resides in the use of an agent for increasing vascular permeability within the cervix in the manufacture of a medicament for hastening cervical ripening, wherein the agent is selected from the group consisting of vascular endothelial growth factor (VEGF), VEGF₁₂₁, VEGF₁₄₅, VEGF₁₆₅, VEGF₁₈₉, VEGF₂₀₆, a smaller, active VEGF fragment, and placenta growth factor (PIGF).

In another of its aspects, the present invention resides in the use of an agent which reduces vascular permeability in the manufacture of a medicament for delaying cervical ripening, wherein the agent is selected from the group consisting of VEGF receptor antagonists, VEGF receptor antibodies, agents which decrease the expression of VEGF, agents which neutralise the effect of VEGF, PIGF receptor antagonists, PIGF receptor antibodies, agents which decrease the expression of PIGF, agents which neutralise the effect of PIGF, soluble VEGF receptors, bradykinin receptor antagonists and histamine receptor antagonists.

Our studies have revealed that VEGF mRNA is highly expressed in human cervix and that VEGF peptide is localised around cervical blood vessels, thereby indicating that VEGF plays an important role in cervical ripening.

VEGF, a heparin binding growth factor with a molecular weight of 45 kDa, is a dimeric secreted glycoprotein composed of two identical subunits linked by disulphide bonds which promote vascular endothelial cell growth. Due to the alternative splicing of mRNA, five different molecular species of VEGF are generated. These VEGFs have 121 amino acids (VEGF₁₂₁), 145 amino acids (VEGF₁₄₅), 165 amino acids (VEGF₁₆₅), 189 amino acids (VEGF₁₈₉) and 206 amino acids (VEGF₂₀₆). VEGF exhibits 18% overall sequence homology with platelet-derived growth factor and 53% sequence homology with placenta growth factor (PIGF). VEGF is not only a powerful mitogen for endothelial cells, but also mediates a number of other endothelial effects, including secretion of collagenase IV, nitric oxide (NO), urokinase-type plasminogen activating factors and PAI-1. In addition to the endothelial mitogenic capacity of the VEGF family, VEGF₁₈₉ is known to induce fluid and protein extravasation from blood vessels. On a molar basis, VEGF is 5 x 10³ times more potent than histamine at increasing vascular permeability. Whilst there is evidence that VEGF is present in the ovary, in human endometrium and in human placenta, as far as we are aware it has never previously been identified in the cervix.

The agent for hastening cervical ripening may be VEGF₁₂₁, VEGF₁₄₅, VEGF₁₆₅, VEGF₁₈₉ and/or VEGF₂₀₆, or a smaller, active VEGF fragment, or another compound of the VEGF family, eg PIGF. PIGF is available from R & D Systems Ltd, Nottingham, GB.

It has been found that VEGF increases connexin-43 and so may be used in the manufacture of medicament for promoting labour by increasing connexin-43. This is described hereinafter in greater detail. Thus, VEGF may be administered concomitantly with oxytocin, especially in cases where myometrial contractility is weak, to promote labour.

In the case where cervical ripening is to be delayed, the agent is one which reduces vascular permeability. Such an agent may be selected from VEGF receptor antagonists, VEGF receptor antibodies, agents which decrease the expression of VEGF, agents which neutralise the effect of VEGF, PIGF receptor antagonists, PIGF receptor antibodies, agents which decrease the expression of PIGF, and agents which neutralise the effect of PIGF.

The body also produces endogenous soluble receptors for VEGF such as in the case of fibroblast growth factor (FGF). These soluble VEGF receptors, such as kinase domain receptor (KDR/flk-1) and flt-1 (fms-like tyrosine kinase) receptor will block the effect of VEGF and can serve as antagonists in delaying labour.

It is considered that suitable VEGF receptor antagonists can be found amongst the antibodies raised against VEGF. The following serve as examples:-
VEGF mAb A4.6,1 Genentech, USA (Growth Factors 7:53-64, 1992).
VEGF (A-20) sc-152 (Santa Cruz Biotechnology, USA)
fit (C-17) sc-316 (Santa Cruz Biotechnology, USA)
flk-1 (C-20) sc-315 (Santa Cruz Biotechnology, USA)
flt-4 (C-20) sc-321 (Santa Cruz Biotechnology, USA)
PIGF antibody

Antisense oligonucleotides which block VEGF expresssion, or PIGF expression or VEGF/PIGF receptors are also considered to be suitable for delaying cervical ripening.

Bradykinin B₂ receptor antagonists such as Hoe-140 (Hoechst AG, Germany) and histamine receptor antagonists may also delay labour.

### Brief Description of the Drawings

The present invention will now be described in further detail and with reference to the accompanying drawings, in which:-
Figure 1A shows products separated by agarose gel electrophoresis of reverse transcriptase-polymerase chain reaction (RT-PCR) amplification of cDNA generated from various cervical tissue samples,
Figures 1B and 1C are autoradiographic pictures of amplified products shown in Figure 1 which have been hybridised with a VEGF-specific probe (Fig. 1B) or a G3PDH specific probe (Fig. 1C) labelled with γ³² P ATP,
Figures 2A and 2B are respectively light and dark field autoradiographs showing the expression of VEGF mRNA in the basal laminar of crypts in a cervical tissue sample,
Figures 2C and 2D are respectively light and dark field autoradiographs showing the expression of VEGF mRNA in the periglandual stroma in a cervical tissue sample,
Figure 3A is an autoradiograph showing staining to VEGF in the vascular smooth muscle layer of cervical blood vessels (bv) in a cervical tissue sample which had been treated with a rabbit polyclonal antibody raised against human VEGF as a primary antibody followed by amplification using a goat anti-rabbit secondary antibody,
Figure 3B is a comparative autoradiograph obtained using normal rabbit serum instead of the primary, VEGK-specific rabbit antibody used for Figure 3A,
Figure 4A is an autoradiographic picture obtained upon Northern blot hybridisation showing the effect of the ovarian steroid hormones, 17-β Estradiol (E₂) and Progesterone (P₂), at various concentrations on VEGF₁₆₅ mRNA in non-pregnant cervix,
Figure 4B is an autoradiographic picture obtained upon Northern blot hybridisation showing, for comparison of RNA loadings, an 18s rRNA oligonucleotide probe hybridised to the same filter,
Figure 4C is a bar chart showing VEGF₁₆₅/18s mRNA ratios for the various concentrations of the ovarian steroid hormones used for Figure 4A, Figure 5A and 5B are Western blots showing the effect of the ovarian steroid hormones, 17-β Estradiol (E₂) and Progesterone (P₂), at various concentrations on VEGF protein expression in media of non-pregnant cervical explants,
Figures 6A to 6H are autoradiographs showing high localisation of VEGF in the artery, vein, capillary and crypt of the pregnant cervix as compared to the non-pregnant cervix,
Figure 7 is a bar chart showing the effects illustrated in Figures 6A to 6H, Figures 8A and 8B are Western blots showing the effect of hrVEGF₁₆₅ and the ovarian steroid hormones, 17-β Estradiol (E₂) and Progesterone (P₂), at various concentrations on Connexin-43 expression in non-pregnant myometrial (FIG. 8A, n = 2) and non-pregnant cervical (FIG. 8B, n = 2) explants,
Figures 9A and 9B are Western blots showing brain cNOS (Fig. 8A) and endothelial cNOS proteins (Fig. 8B) from human myometrium collected after elective Caesarean section (ie non-labouring myometrium, Group 1, n=3) and emergency Caesarian section (ie labouring myometrium, Group 2, n =2), both at term delivery.

### Detailed Description of the Invention

### MATERIALS AND METHODS

### Tissue collection and process

Full thickness cervical biopsies were collected from women of reproductive age undergoing hysterectomy for the investigation of subjective menorrhagia (non-pregnant) and gravid hysterectomy (pregnant cervix). Tissue obtained from such women was shown to be histologically normal. For immunohistochemical studies, the tissue was rinsed in sterile saline and immediately immersed in 10% formaldehyde and processed for paraffin wax embedding. A human cervical carcinoma epithelial cell line, Caski, was also used in these studies.

For immunohistochemical studies, the tissue was rinsed in sterile saline and immediately immersed in 10% formaldehyde and processed for paraffin wax embedding. For RT-PCR studies, total RNA was extracted from 200 mg of frozen pieces of cervical tissue and from one flask of Caski cells, by the RNAzol B method (AMS Biotechnology, Witney, Oxon, UK). Briefly, tissue was homogenised in a buffer containing RNAzol and total RNA was extracted by a single step procedure of the acid-guanidinium thiocyanate-phenol-chloroform method (Chomczynski and Sarchi, Analytical Biochem., 162:156-159, 1987). For *in situ* hybridization studies, frozen tissues were surrounded in embedding medium (OCT compound, Miles Scientific) before 10 µm sections were cut using a cryostat (-15 to -19°C) and thaw mounted onto poly-L-lysine (Sigma, Poole, U.K.) coated glass slides. Section were stored (less than 2 weeks) at -70°C until being prepared for *in situ* hybridization.

### Pre-Capillary Quantitation Study in Human Non-Pregnant Cervix

The aim of the research was to quantify the pre-capillary density in four histological differentiated parts of the human uterine non-pregnant cervix. The pre-capillary density in four histological differentiated parts of the cervix was measured with a microscope in serial 5 µm tissue sections obtained from cone biopsies fixed in formalin and embedded in paraffin wax. The sections stained with QBend10 using the ABC/HRP labelling method. The pre-capillary density was taken at an objective magnification of x10. The parts of the uterine cervix to be examined quantitatively were established as follows:-
(a) the side margins corresponding to the area of stroma just underneath the squamous epithelium;
(b) the one just underneath the columnar epithelium;
(c) the deep margin corresponding to a deep stroma area; and
(d) the margin corresponding to the transformation zone of the uterine cervix.

Since the QBend10 itself stains the endothelial lining, the boundaries external to this in each single capillary were hand-drawn interactively on a defined dimensions area on a paper.

### RT-PCR

cDNA was synthesised according to the manufacturer's instructions by using a cDNA Cycle Kit (Invitrogen) which makes use of AMV reverse transcriptase to generate high yields of full-length first-strand cDNA from RNA for use as a template in polymerase chain reaction (PCR) amplifications. After first strand synthesis, 10% of the reverse transcriptase reaction was added to 10mM Tris-HCI, pH 8.8, 1.5mM MgCl₂, 50mM KCI, 0.1% v/v Triton-X100, 125mM dNTPs, 1mM of each primer and DynaZyme Thermostable DNA Polymerase (2U; Finnzyme Oy Espoo, Finland). Antisense VEGF primer or Oligo -dT primer was used for priming the RNA. The internal standard for quantifying gene expression was glyceraldehyde 3-phosphate dehydrogenase (G3PDH). The sequence of the primers used in this study are shown below.

### G3PDH:

Sense (5') primer: 5' TGAAGGTCGGAGTCAACGGATTTGGT 3' Antisense (3') primer: 5' CATGTGGGCCATGAGGTCCACCAC 3'

### VEGF

Sense (5') primer: 5' GAAACCATGAACTTTCTGCTG 3'

Antisense (3') primer: 3' TGTATCAGTCTTTCCTGGTGA 3'

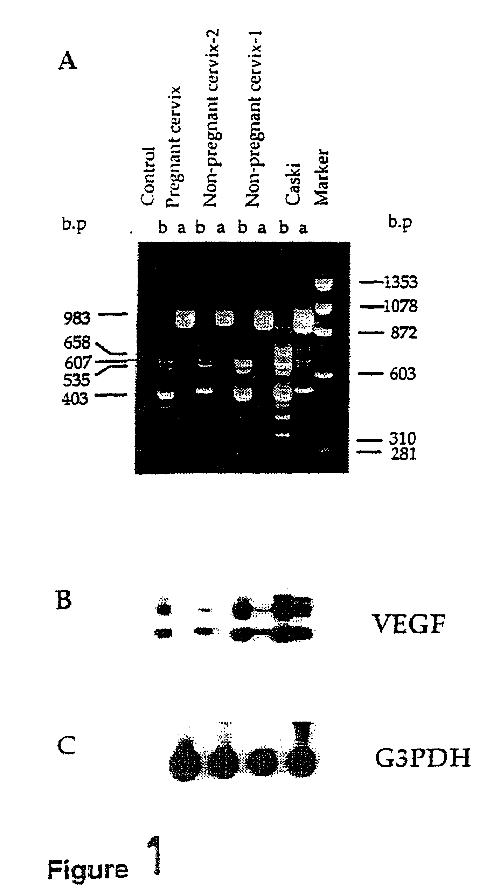

Amplification cycles were 1 min at 95°C, 2 min at 55°C and 3 min at 72°C in a 480 Perkin Elmer Cetus DNA Thermal Cycler. The amplified products were separated by 1.2% agarose gel electrophoresis (Fig. 1A) and transferred to a positively charged Hybond-N⁺ membrane by one hour downward capillary transfer (Chomczynski and Sarchi, Analytica Biochem.,201:134-139, 1992) and hybridised with a VEGF specific probe (Fig. 1 B) and a G3PDH probe (Fig. 1C) labelled with γ³²P ATP. Hybridisation was performed overnight at 42°C in 6x Net, 5x Denhart's, 0.5% Nominet P-40, 10% Dextran and 100 mg/ml heat-denatured salmon sperm DNA. The hybridised membrane was washed to a final stringency of 2x SSC (standard saline citrate) and 0.1% SDS (sodium dodecyl sulphate) at 42°C and exposed to autoradiography at -70°C for 1 day.

### In situ hybridization

### Pre-treatment of Sections

The poly-L-lysine coated sections were fixed in 4% paraformaldehyde in PBS (pH 7.4) for 5 min, acetylated in 0.1 M triethanolamine (TEA), pH 8.0, with acetic anhydride (0.25% w/v) for 10 minutes. Slides were dehydrated through an ascending graded series of alcohol (70%, 80%, 95% and 100% v/v) followed by incubation in chloroform for 5 min. The sections were then partially rehydrated sequentially in 100% (v/v) and 95% (v/v) ethanol and air dried. As a control for non-specific binding, before fixation, some sections were treated with 100 µgm/ml of RNAse A (Sigma, Poole, U.K.) for 1 hour at 37°C.

### Preparation of Probes

VEGF Probe: The probe for VEGF was a synthetic oligonucleotide probe directed against human VEGK cDNA (21 bases directed against base pairs 874 to 853 of human VEGF cDNA) which had been tail-labelled with [³⁵S] dATP for in situ hybridization by 3' terminal deoxynucleotidyl transferase using a commercial available kit (NEN - DuPont). As a control, the sense probe (containing the same sequence as the human VEGF cDNA) was also end labelled with [³⁵S] dATP by the same method as above.

Hybridisation: Following acetylation, the air dried slides were hybridised in a moist chamber under coverslips in a hybridization buffer containing formamide (50% v/v), 3 M NaCI, 0.3 M Na citrate, 0 1 mM EDTA, dextran sulphate (10% w/v), 0.5ml Denhardt's reagent, 100 µgm/ml of salmon sperm DNA, 100 µgm/ml of yeast transfer RNA, 100 µgm/ml of polyadenylic acid A and 1 x 10⁶ d.p.m. /ml of ³⁵S labelled sense or antisense RNA probe. The sections were covered with a paraffin coverslip and hybridisation was carried out in a moist chamber at 50°C for 16 hours. Following hybridisation, the coverslips were removed in a large volume of 1xSSC, the sections were then washed three times in 1xSSC at wash temperature for 20 minutes, then twice for 60 minutes at room temperature and briefly rinsed in distilled water. At least three consecutive sections were used for hybridization from each cervix, two were labelled with the antisense probe and one was with the sense probe. Autoradiography was carried out by coating the slides in Ilford K5 emulsion (Ilford Ltd. Cheshire, UK). Following dilution of the emulsion (1:1) in distilled water with 2% (v/v) glycerol, the slides were dipped in the emulsion, dried on the bench for at least two hours and the dry slides were stored in a light-proof box containing silica gel at 4°C for 3 weeks. The sections were then stained lightly with 0.1% cresyl violet and mounted.

Hybridization: Following acetylation, the air dried slides were hybridised in a moist chamber under coverslips in the hybridization buffer containing formamide (50% v/v), 3 M NaCI, 0.3 M Na citrate, 0 1 mM EDTA, dextran sulphate (10% w/v), 0.5 ml Denhardt's reagent, 100 µgm/ml of salmon sperm DNA, 100 µgm/ml of yeast transfer RNA, 100 µgm/ml of polyadenylic acid A and 1 x 106 d.p.m./ml of ³⁵S labelled sense or antisense RNA probe. Hybridisation was carried out at 50°C for 16 hours. The slides were then washed in 4x standard saline citrate (SSC) and then treated with 20 µg/ml RNAse A (Sigma, Poole, UK.) for 20 minutes at 37°C in 0.5M NaCI, 10mM Tris pH 7.0, 1mM EDTA. This was followed by washes of increasing SSC stringency. The slides were washed twice in 2 x SSC with 1M dithiotheritol (DTT) for 10 minute at room temperature followed by 1 x SSC with 1M DTT for 20 minutes, and then washed at 0.5 x SSC with 1M DTT for 20 minutes, and finally in 0.1 x SSC with 1M DTT for 60 minutes at 65°C and dehydrated in an ethanol series.

### Immunocytochemistry

Serial 3 µm sections of formalin-fixed, paraffin-embedded tissue prepared as described above were used for immunohistochemistry. These sections were de-paraffinized by incubation for 5 min with Histoclear and hydrated in methanol, and endogenous peroxidase activity was quenched by the addition of 0.3% (v/v) of hydrogen peroxide for 10 minutes. The primary antibody was a rabbit polyclonal antibody raised against the human VEGF and was purchased from Peninsula Laboratories, Merseyside, UK. It is highly specific but cross reacts completely with human VEGF. Nonimmune goat serum (10% in 0.05 mol/l PBS) was used as a dilution of the primary antibody to reduce non-specific binding. Amplification of the primary antibody reaction was achieved using a goat anti-rabbit secondary antibody (diluted 1:200 in 0.05 mol/l PBS, pH 7.4) for 30 min followed by a complex of streptavidin (Dako Ltd, Bucks, UK) and biotinylated peroxidase (Dako Ltd, Bucks, UK). Finally, the binding was visualised by the addition of 0.5 mg/ml diaminobenzidine (Sigma Chemical Co. Ltd, Poole, Dorset, UK) and 0.01% hydrogen peroxide in 0.05 mmol/l PBS to the antigen-antibody complex. Between each step, the sections were washed in 3 x 200 ml of 0.1% (v/v) polyoxylene-10-oleoyl-ether in 0.05 mmol/l PBS over a period of 15 min. All incubations of antisera were carried out at a room temperature in a wet chamber mounted on a rocking tray which ensures a movement of antiserum over the whole section. Then the sections were counter-stained with Mayers Haematoxylin, dehydrated and mounted.

To test the specificity of the immunohistochemical staining, the primary antibody was omitted from the sections, or replaced with goat nonimmune serum in control experiments.

### RESULTS AND DISCUSSION

### Quantitation of pre-capillary staining in human non-pregnant cervix

The following table shows the results of the quantitative evaluation of the capillary density in the stroma of the uterine cervix:-

| **AREA OF CERVIX** | **CAPILLARY DENSITY (c/mm**^{**3**}**)** |
|---|---|
| Squamous epithelium | 13 |
| Deep stroma | 11 |
| Transformation zone | 4 |
| Columnar epithelium | 3 |

In the stroma area underneath the squamous epithelium, the value of the capillary density or number of capillaries per square mm is 13. Similar capillary density values (11) can also be seen in the deep layers of stroma. The values corresponding to capillary density become lower though, from the area of stroma close to the transformation zone (4) up to the area of stroma close to the columnar epithelium (3). These areas of stroma having the lowest capillary density values were shown to have a high density of crypts.

### VEGF mRNA expression

Total RNA from non-pregnant cervix was reverse transcribed and subjected to 30 rounds of amplification of PCR using VEGF sense and an antisense oligonucleotide primer shared by all differentially spliced VEGF mRNA species. Hybridisation using an antisense VEGF probe showed that the amplified species, identified in all tissues examined, were 403, 535, 607 and 658 bp fragments corresponding to the mRNA encoding VEGF₁₂₁, VEGF₁₆₅, VEGF₁₈₉ and VEGF₂₀₆, respectively (Figure 1B). However, the transcript corresponding to VEGF₂₀₆ was shown to exist in lower amounts, while the VEGF₁₂₁ transcript was found to exist in higher amounts among the other VEGF transcripts in each one of the samples tested. A control reaction without input cDNA gave no product, thus eliminating the possibility of contamination. The cervical carcinoma cell line was also found to express the same four transcripts of VEGF mRNA.

### VEGF mRNA localization

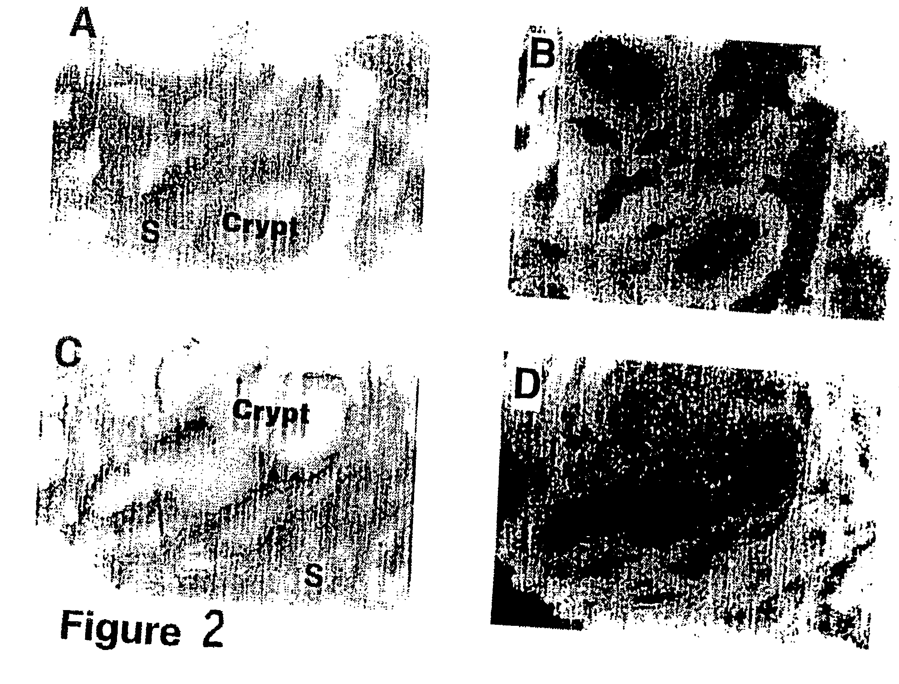

In order to identify the site of expression of VEGF mRNA, *in situ* hybridisation was carried out on samples of cervix. The expression of VEGF mRNA was demonstrated in the basal laminar of crypts and in the periglandual stroma (Figure 2). There was an apparent lack of hybridisation signal in the epithelial cells of the crypts.

### VEGF peptide localization

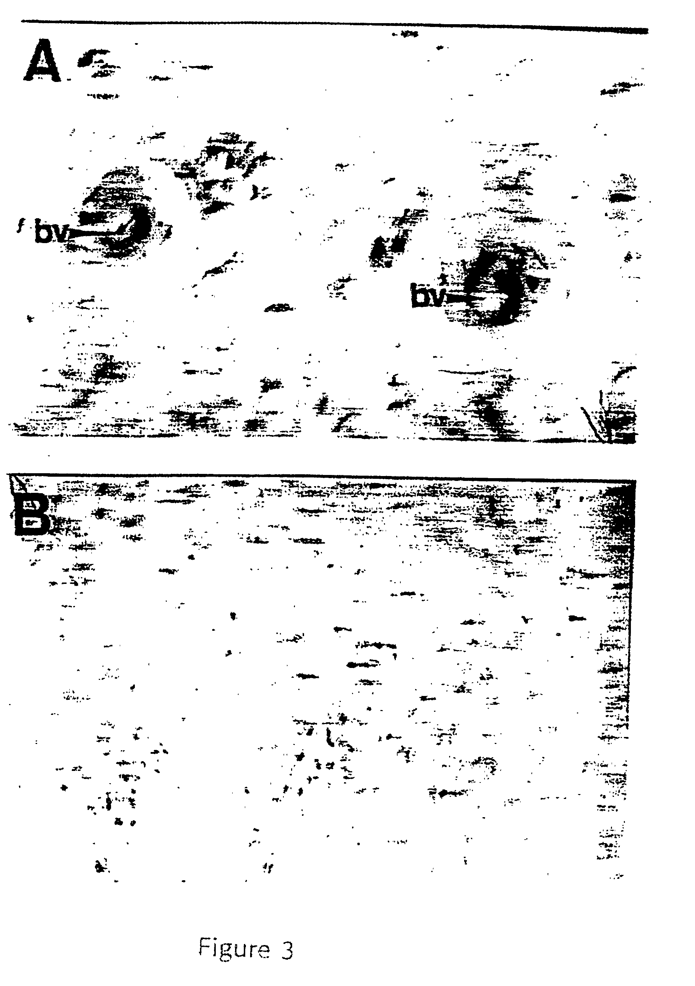

Strong VEGF immunoreactivity was detected in vascular smooth muscle layer of the cervical blood vessels and a diffuse staining was seen in some elements of the cervical connective tissue such as fibroblasts and mast cells (Fig 3A). Cervical glands and the crypts showed no staining to VEGF (data not shown). In control sections, no staining was seen using normal rabbit serum instead of primary antibody (Fig 3B).

Further experimental work in relation to the present invention is described below:-

### Source of tissue:

Human uterine non-pregnant cervix tissue was obtained from nonmalignant hysterectomies. The samples were maintained in phenol red free culture media DMEM supplemented with penicillin (1%), streptomycin (1%) and L-Glutamine (1%) and transferred to the laboratory on ice. The biopsies were washed in ice-cold saline solution then cut into small sections 1 to 2mm and incubated in the above culture medium in 60 x 15 mm petri dishes at 37°C in 5% CO₂/95% air. Progesterone (P₂) was added to the culture medium at concentrations of 10⁻⁴, 10⁻⁶ and 10⁻⁸ M. 17-β Estradiol (E₂) was added to the culture medium either alone at concentrations of 10⁻⁶, 10⁻⁷ and 10⁻⁸M or for 2 hours to prime the cervical explants at a concentration of 10⁻⁷M, followed by addition of Progesterone (P₂) at concentrations ranging from 10⁻⁴ to 10⁻⁸M. The effect of VEGF₁₆₅ (1, 10, 50, 100, 200 ng/ml) in the non-pregnant cervical explants was also investigated. This culture system was chosen because it allows study of the intact tissues in their own matrix and avoids the potentially artefact-producing procedures of cell dispersion and culture. In this system, the cells remain intact for at least 5 days.

### Western Blot Analysis:

Cervical tissue, after having been stimulated with VEGF₁₆₅ for 24 hours or with the ovarian steroid hormones, 17β-estradiol (E₂) and progesterone (P₂), for 48 hours, was washed twice in ice-cold PBS and homogenised in ice-cold, high salt lysis buffer (containing 0.4M KCI, 20 mM HEPES, 1mM DTT, 20% glycerol, 0.5mg/ml Bacitracin, 40 µg/ml PMSF, 5 µg/ml Peptastin, 5 µg/ml Leupeptin). Homogenates were centrifuged at 12,000 revolutions / min for 15 minutes and the supernatants containing extracted protein were collected. The culture medium used to incubate the tissue with or without E₂, P₂ or E₂+P₂ was also collected, centrifuged. To the supernatant, 3 volumes of ethanol were added and stored at -70°C for at least 5 hours. Then, the culture medium/ethanol suspension was centrifuged at 10,000 rpm for 10 min and the pellet containing the protein was resuspended in lysis buffer. Total protein, extracted from both tissue and medium, was quantified with the Bio-Rad protein assay, and 500 ng of protein from each sample was diluted in loading buffer (containing 0.0 Tris-HCI, 0.002 M EDTA, 2% SDS, 10% mercaptoethanol, 20% glycerol, bromophenol blue). Samples were subjected to electrophoresis in a 12% polyacrylamide gel with 5% stacking gel and transferred overnight at 36V onto a nitrocellulose membrane. The membrane was blocked at RT with 1% skimmed milk in Tris-buffered saline solution before incubation with an antiserum (diluted 1:500) directed against the rabbit VEGF₁₆₅ at 4°C. After a 15 min. wash and 2x5 min. washes with Tris-buffered saline solution at room teperature (RT), the membrane was incubated in an anti-rabbit secondary Ab conjugated to alkaline phosphatase. In case of Western blotting experiments for Connexin-43, the membrane was blocked overnight at 4°C in blocking buffer (1% BSA in 10mM Tris pH 7.5, 100 mM NaCl, 0.1% Tween 20), before incubation with a monoclonal antibody directed against Connexin-43 again overnight at 4°C. After a 6x5 min. washes with Tris-buffered saline solution, the membrane was incubated in a mouse secondary Ab conjugated to alkaline phosphatase. A chemiluminescence system (ECL-Amersham) was used to visualise the presence of VEGF₁₆₅, Connexin-43 or Flt-1 protein. This is a highly sensitive and quantitive assay for detecting proteins immobilised on membranes.

### Northern Blot Hybridisation:

RNA (40µg/lane) was loaded onto a 1.2% agarose/formaldehyde gel, electrophoretically separated, transferred onto Hybond nylon membranes, and immobilised by exposure to U.V. light for 5 min. Membranes were prehybridized at 42°C for 8 hours in a solution containing 50% formamide, 5x SSC, 5x Denhardt's solution, 0.5% SDS and salmon sperm DNA at 150 µg/ml. The VEGF₁₆₅ cDNA was labelled with [α-³²P]dCTP by random-primer extension. 18s oligonucleotide probe labelled by T₄ polynucleotide kinase with [γ³²P]ATP was used to determine the relative amount of RNA loaded in each lane by rehybridizing the blots that have previously been probed with VEGF₁₆₅. Hybridisation was done under the same conditions with the labelled probe for 18 hours at 42°C. The membrane was washed at 1xSSC, 0.1%SDS for 15 min at RT, in 0.5x SSC, 0.1% SDS for 15 min at RT, and exposed to an intensifying screen cassette at -70°C for 2 days.

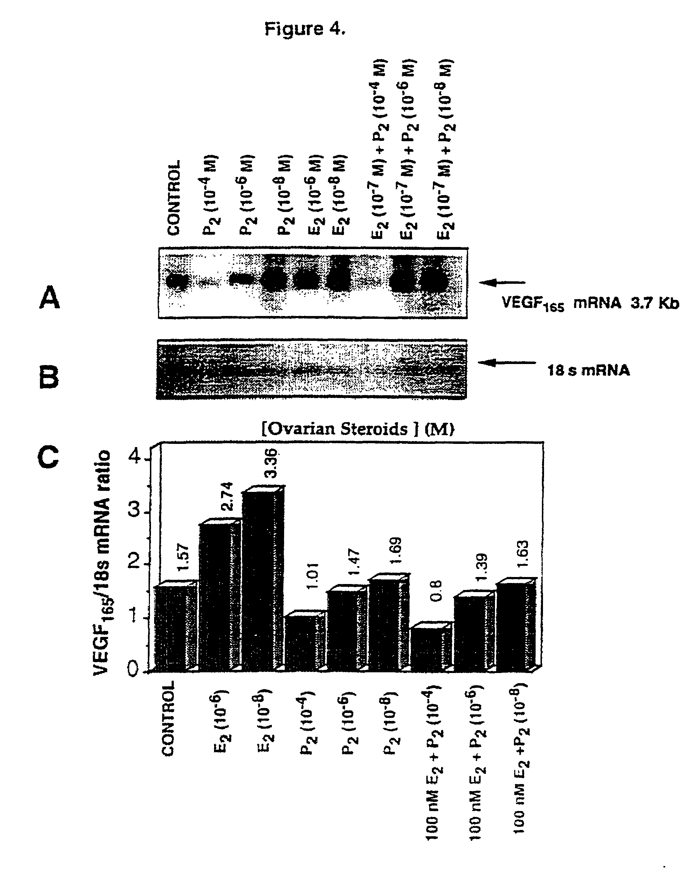

Cervical tissue explants in culture medium were exposed to various concentrations of the ovarian steroid hormones, 17β-estradiol (E₂) and progesterone (P₂), for 48 hours, followed by extraction of mRNA from the tissue. Northern blot analysis shows that 17β-estradiol up-regulates VEGF compared to Control, whereas progesterone at 10⁻⁴ M down-regulated VEGF mRNA as did progesterone at 10⁻⁴ M in the presence of 10⁻⁷ M 17β-estradiol (Figure 4A). For comparison of RNA loading, an 18s rRNA oligonucleotide probe was hybridised to the same filter (Figure 4B). The above up-regulation and down-regulation effects are shown in the bar chart of the densitometric analysis of VEGF abundance relative to 18s mRNA (Figure 4C).

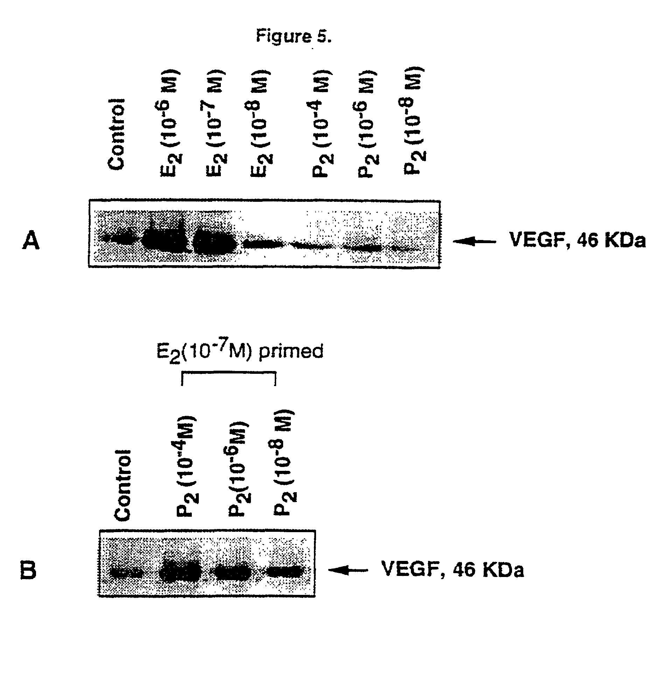

Cervical tissue explants in culture medium were exposed to various concentrations of the ovarian steroid hormones, 17β-estradiol (E₂) and progesterone (P₂), for 48 hours, followed by extraction of protein from the tissue as well as the culture medium. Western blot analysis shows that 17β-estradiol up-regulates the secreted forms of VEGF in the culture medium. In the tissue itself, 17β-estradiol had no effect, suggesting that the secreted isoforms were affected by 17β-estradiol. In contrast, progesterone at 10⁻⁴ M down-regulated VEGF both in the culture medium and in the tissue itself as compared with the Control. (Figure 5A).

When the *in vivo* situation was mimicked by priming the cervical explants with 17β-estradiol for 4 hours prior to addition of progesterone, the increasing concentration of progesterone caused a dose-dependent increase in VEGF protein expression both in the culture medium and the tissue. (Figure 5B).

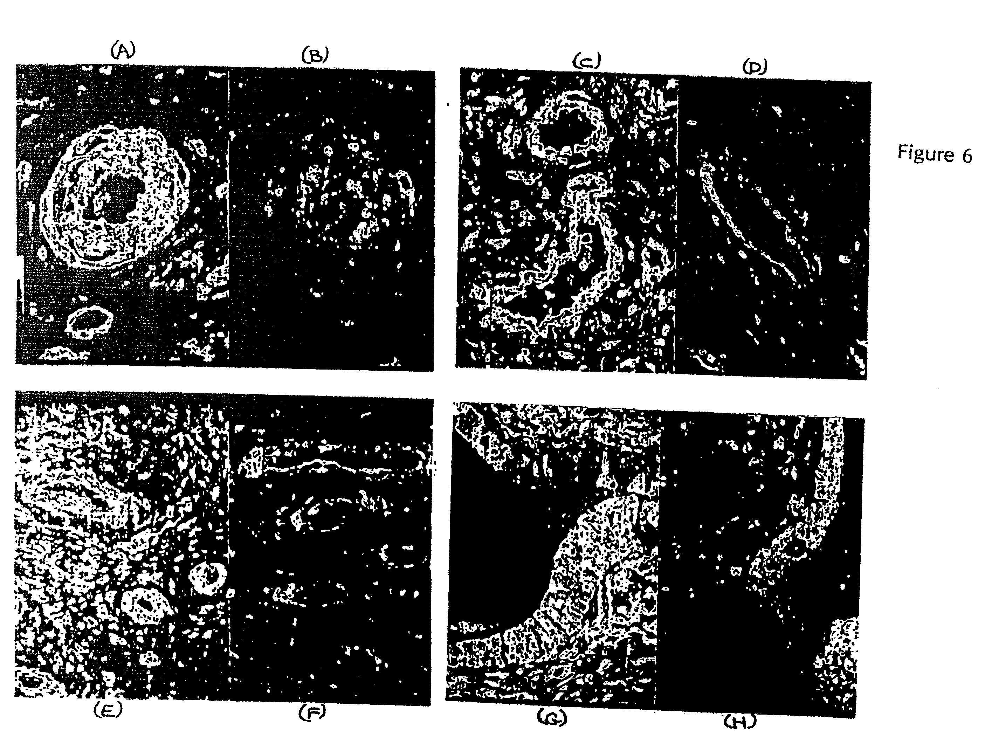

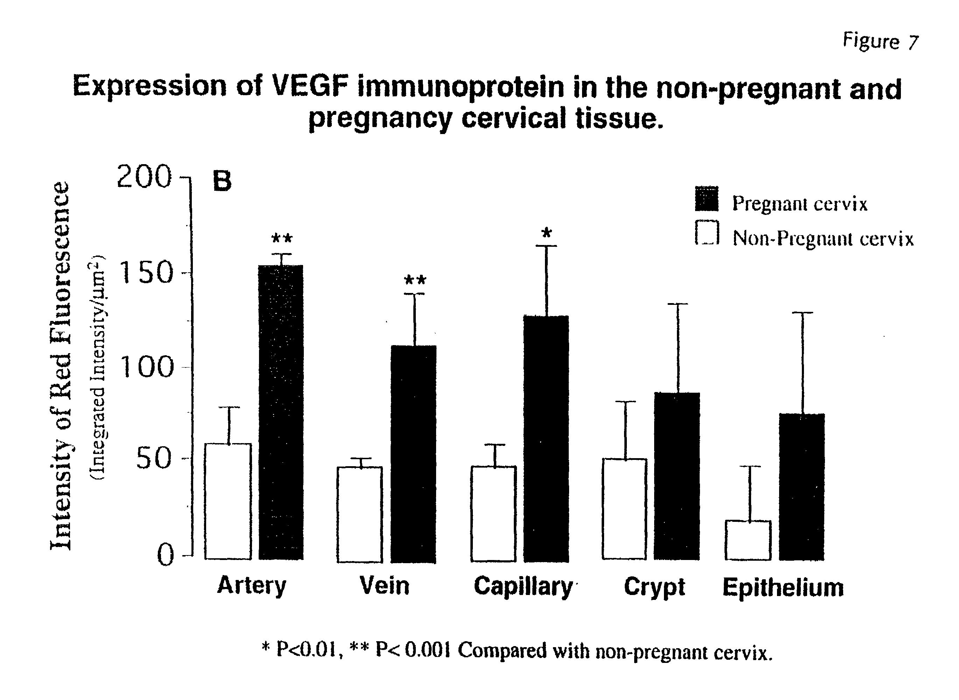

Using a fluorescent label, alkaline phosphatase-Fast Red method, we have shown that VEGF is highly significantly localised in the media of pregnant cervical blood vessels as compared to the non-pregnant cervix. The red reaction product of alkaline phosphatase immuno-conjugates produces a brilliant red fluorescence that is visible by fluorescence microscopy using both fluorescent and rhodamine filter combinations. The fluorescence properties of the reaction product allows quantitation by image analysis using confocal microscopy. Figures 6A and 6B show an artery in pregant and non-pregnant cervix, respectively. Figures 6C and 6D show a vein in pregant and non-pregnant cervix, respectively. Figures 6E and 6F show a capillary in pregant and non-pregnant cervix, respectively. Figures 6G and 6H show a crypt in pregant and non-pregnant cervix, respectively. These effects are graphically shown in Figure 7 which also includes comparative data on the epithelium in pregnant and non-pregnant cervical tissue.

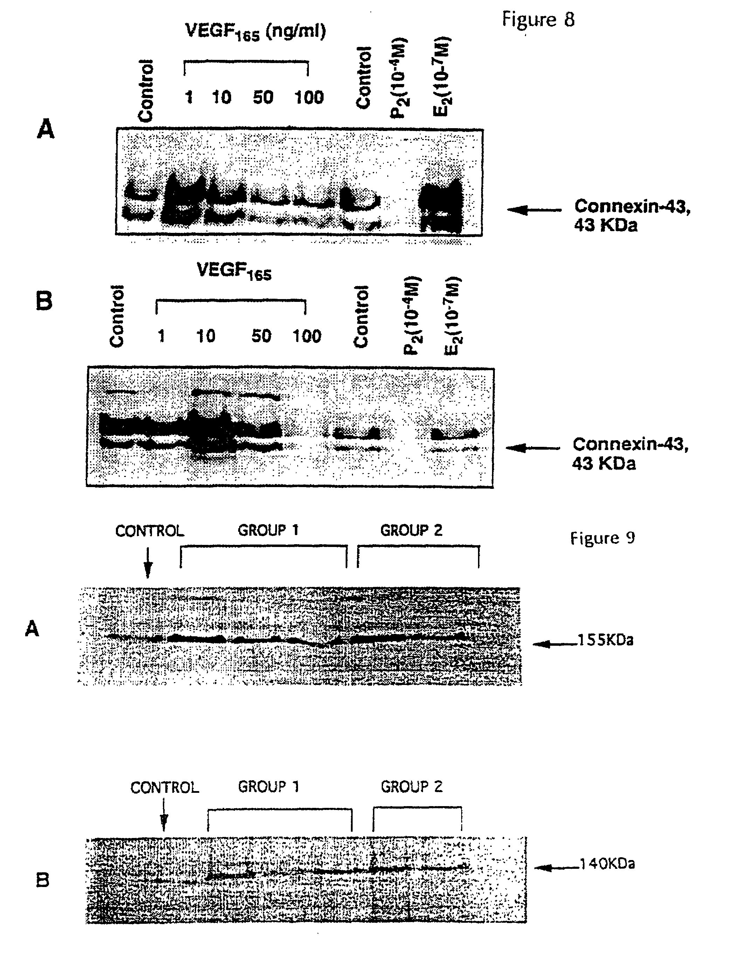

Figures 8A and 8B respectively show the up-regulation of connexin-43 expression by VEGF in myometrial and cervical explants. Gap junctions are proteinaceous pores connecting cells which allow passage of ions and other small molecules from one cell to another, allowing cell-to-cell communication. Tyrosine phosphorylation of connexin-43, a 43kDa isoform of gap junction protein, has been shown to inhibit transport through the gap junction. In late pregnant guinea pigs, increase in the density of gap junctions as demonstrated immunocytochemically by a dramatic increase in connexin-43 in the myometrium following treatment with antiprogestins, supports the role of these structures as sites of propagation and the basis for synchrony during labour. VEGF may promote labour as well as induce cervical ripening by increasing cell-to-cell communication.

Nitric oxide is believed to maintain a quiescent state in the myometrium during pregnancy and labour, and animal studies have shown that, at the time of labour, nitric oxide levels drop to allow increased myometrial cell activity. We have shown that there is no difference in nitric oxide synthase (NOS) immunoreactivity between labouring and non-labouring myometrium by ECL Western blot analysis (Figure 9). We have shown that nitric oxide donors decrease and inhibitors of NOS increase both

VEGF in the cervical explants, and thus it is considered that the use of a nitric oxide donor, e.g. glycerol trinitrate (GTN), can inhibit cervical ripening by decreasing VEGF expression. A nitric oxide donor, e.g. GTN, can be administered intravaginally, according to our proposal to delay the onset of labour.

### Compositions for different patient treatment regimes

The invention proposes to prepare compositions to treat patients in one of the following ways:-
(1) Instillation directly into the cervical canal or intravaginally using vehicles such as water-soluble gels or slow-releasing resins.
(2) Intravenous infusion, which may be the preferred method of treatment.
(3) As a pellet inserted directly under the skin using a long-acting, near-zero order releasing system.
(4) By oral administration of the drug.

## Claims

1. The use of an agent for increasing vascular permeability within the cervix in the manufacture of a medicament for hastening cervical ripening, wherein the agent is selected from the group consisting of vascular endothelial growth factor (VEGF), VEGF₁₂₁, VEGF₁₄₅, VEGF₁₆₅, VEGF₁₈₉, VEGF₂₀₆, a smaller, active VEGF fragment, and placenta growth factor (PIGF).

2. The use of an agent for increasing vascular permeability within the cervix in the manufacture of a medicament hastening cervical ripening, wherein the agent is one which increases the expression of VEGF.

3. The use as claimed in claim 2, wherein the agent is a nitric oxide synthase inhibitor.

4. The use of an agent which reduces vascular permeability in the manufacture of a medicament for delaying cervical ripening, wherein the agent is selected from the group consisting of VEGF receptor antagonists, VEGF receptor antibodies, agents which decrease the expression of VEGF, agents which neutralise the effect of VEGF, PIGF receptor antagonists, PIGF receptor antibodies, agents which decrease the expression of PIGF, agents which neutralise the effect of PIGF, soluble VEGF receptors, bradykinin receptor antagonists and histamine receptor antagonists.

5. The use as claimed in claim 4, wherein the agent is a VEGF receptor antagonist and is selected from the group consisting of VEGF mAb A4.6,1, VEGF (A-20) sc-152, fit (C-17) sc-316, flk-1 (C-20) sc-315, flt-4 (C-20) sc-321.

6. The use as claimed in claim 5, wherein the agent is one which decreases the expression of VEGF and is a nitric oxide donor.

7. The use of VEGF in the manufacture of a medicament for promoting labour by increasing connexin-43.

## Revendications

1. Utilisation d'un agent pour augmenter la perméabilité vasculaire dans le col de l'utérus, lors de la fabrication d'un médicament pour accélérer le mûrissement cervical, dans laquelle l'agent est choisi dans le groupe constitué par le facteur de croissance endothéliale vasculaire (VEGF), VEGF₁₂₁, VEGF₁₄₅, VEGF₁₆₅, VEGF₁₈₉, VEGF₂₀₆, un fragment de VEGF plus petit actif, et le facteur de croissance du placenta (PIGF).

2. Utilisation d'un agent pour augmenter la perméabilité vasculaire dans le col de l'utérus, lors de la fabrication d'un médicament accélérant le mûrissement cervical, dans laquelle l'agent est un agent qui augmente l'expression de VEGF.

3. Utilisation selon la revendication 2, dans laquelle l'agent est un inhibiteur de l'oxyde nitrique synthase.

4. Utilisation d'un agent qui réduit la perméabilité vasculaire, lors de la fabrication d'un médicament pour rertarder le mûrissement cervical, dans laquelle l'agent est choisi dans le groupe constitué par les antagonistes des récepteurs de VEGF, les anticorps contre les récepteurs de VEGF, les agents qui diminuent l'expression de VEGF, les agents qui neutralisent l'effet du VEGF, les antagonistes des récepteurs de PIGF, les anticorps contre les récepteurs de PIGF, les agents qui diminuent l'expression de PIGF, les agents qui neutralisent l'effet de PIGF, les récepteurs de VEGF solubles, les antagonistes des récepteurs de bradykinine et les antagonistes des récepteurs d'histamine.

5. Utilisation selon la revendication 4, dans laquelle l'agent est un antagoniste des récepteurs de VEGF et est choisi dans le groupe constitué par VEGF mAb A4.6,1, VEGF (A-20) sc-152, flt (C-17) sc-316, flk-1 (C-20) sc-315, flt-4 (C-20) sc-321.

6. Utilisation selon la revendication 5, dans laquelle l'agent est un agent qui diminue l'expression du VEGF et est un donneur d'oxyde nitrique.

7. Utilisation de VGF lors de la fabrication d'un médicament pour activer le travail en accroissant la connexine-43.

## Patentansprüche

1. Verwendung eines Mittels zum Erhöhen von vaskulärer Permeabilität in der Cervix bei der Herstellung eines Arzneimittels zum Beschleunigen von zervikalem Reifen, wobei das Mittel ausgewählt ist aus der Gruppe, bestehend aus vaskulärem Endothelwachstumsfaktor (VEGF), VEGF₁₂₁, VEGF₁₄₅, VEGF₁₆₅, VEGF₁₈₉, VEGF₂₀₆, einem kleineren, aktiven VEGF Fragment und Plazentawachstumsfaktor (PlGF).

2. Verwendung eines Mittels zum Erhöhen von vaskulärer Permeabilität in der Cervix bei der Herstellung eines Arzneimittels, das zervikales Reifen beschleunigt, wobei das Mittel eines ist, das die Expression von VEGF erhöht.

3. Verwendung nach Anspruch 2, wobei das Mittel ein Stickstoffoxidsynthaseinhibitor ist.

4. Verwendung eines Mittels, das vaskuläre Permeabilität bei der Herstellung eines Arzneimittels zum Verzögern von zervikalem Reifen reduziert, wobei das Mittel ausgewählt ist aus der Gruppe, bestehend aus VEGF Rezeptorantagonisten, VEGF Rezeptorantikörpern, Mitteln, die die Expression von VEGF verringern, Mitteln, die die Wirkung von VEGF, P1GF Rezeptorantagonisten, PlGF Rezeptorantikörpern neutralisieren, Mitteln, die die Expression von PlGF verringern, Mitteln, die die Wirkung von P1GF, löslichen VEGF Rezeptoren, Bradykininrezeptorantagonisten und Histaminrezeptorantagonisten neutralisieren.

5. Verwendung nach Anspruch 4, wobei das Mittel ein VEGF Rezeptorantagonist ist und ausgewählt ist aus der Gruppe, bestehend aus VEGF mAb A4.6,1, VEGF (A-20)sc-152, flt(C-17)sc-316, flk-1(C-20)sc-315, flt-4(C-20)sc-321.

6. Verwendung nach Anspruch 5, wobei das Mittel eines ist, das die Expression von VEGF verringert und ein Stickstoffoxiddonor ist.

7. Verwendung von VEGF bei der Herstellung eines Arzneimittels zum Fördern von Wehen durch Erhöhen von Connexin-43.
